(19) Europäisches Patentamt
European Patent Office
Office européen des brevets

(11) Veröffentlichungsnummer: **0 075 139**
**A1**

# (12) EUROPÄISCHE PATENTANMELDUNG

(21) Anmeldenummer: **82107948.0**

(22) Anmeldetag: **30.08.82**

(51) Int. Cl.³: **C 07 D 307/32**, C 07 D 265/30, C 07 C 103/38, C 07 C 103/48, C 07 C 109/087, C 07 C 109/18, C 07 C 121/30, C 07 C 131/00, C 07 C 133/02, C 07 C 133/04, C 07 C 159/00

(30) Priorität: **05.09.81 DE 3135239**

(43) Veröffentlichungstag der Anmeldung: **30.03.83**
**Patentblatt 83/13**

(84) Benannte Vertragsstaaten: **AT BE CH DE FR GB IT LI NL SE**

(71) Anmelder: **BASF Aktiengesellschaft,**
**Carl-Bosch-Strasse 38, D-6700 Ludwigshafen (DE)**

(72) Erfinder: **Graf, Hermann, Dr., Ginsterstrasse 15, D-6704 Mutterstadt (DE)**
Erfinder: **Zeeh, Bernd, Dr., Thorwaldsenstrasse 5, D-6700 Ludwigshafen (DE)**
Erfinder: **Rentzea, Costin, Dr., Neuenheimer Landstrasse 72, D-6900 Heidelberg (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

## (54) N-substituierte Brenztraubensäureamide, Verfahren zu ihrer Herstellung und diese enthaltende Fungizide.

(57) N-substituierte Brenztraubensäureamide der Formel I

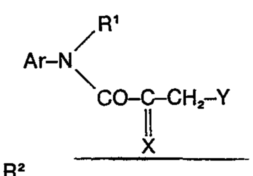
I,

worin

Ar 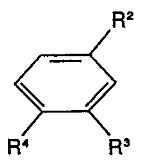 oder 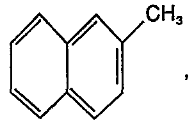,

$R^1$ 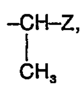, 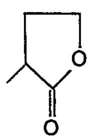, 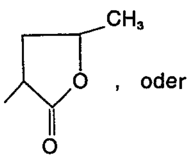, oder $-CH=C(CN)_2$,

oder 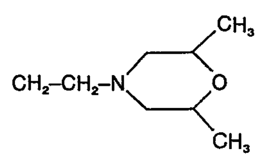

$R^2$ $CH_3$
$R^3$ H, $CH_3$, $C_2H_5$ oder Cl
$R^4$ H, $CH_3$, $C_6H_5$ oder Br

X 0, $N-NR^5R^6$, $N-NH-CO-NR^5R^6$, $N-NH-CS-NR^5R^6$, $N-O-R^7$
Y H oder Br
Z $CO_2CH_3$, $CH(OCH_3)_2$, $CONH-NR^5R^6$, $CO-NH-NH-CS-NR^5R^6$

$R^5$ und $R^6$ unabhängig voneinander H, $CH_3$, $C_2H_5$ oder $C_6H_5$ und
$R^7$ H, $CH_3$, $C_2H_5$, $-CH_2-CH=CH_2$, gegebenenfalls substituiertes Benzyl, $-CH_2-O-CO-CH_3$, $CH_2-CO-O-CH_3$ oder $-CO-CH_3$ bedeutet und diese enthaltende Fungizide.

EP 0 075 139 A1

# N-substituierte Brenztraubensäureamide, Verfahren zu ihrer Herstellung und diese enthaltende Fungizide

Die vorliegende Erfindung betrifft neue aromatische Brenztraubensäureamide, Verfahren zu ihrer Herstellung sowie ihre Verwendung als Fungizide.

Es ist bekannt, Zink-ethylen-1,2-bisdithiocarbamat, das N-Trichlormethylthiophthalimid oder das N-Trichlormethylthiotetrahydro-phthalimid als Fungizide in der Landwirtschaft und im Gartenbau zu verwenden. Die genannten Verbindungen sind gute Mittel zur Bekämpfung von pilzlichen Krankheiten (vgl. R. Wegler, "Chemie der Pflanzenschutz- und Schädlingsbekämpfungsmittel", Band 2, Seiten 65 bis 66 und 109 und Band 4, Seiten 139 und 191, Berlin/Heidelberg/New York, (1970) und (1977)).

Jedoch sind diese Fungizide nicht nach erfolgter Infektion verwendbar und ihre Wirkung bei niedrigen Aufwandkonzentrationen genügt nicht den Anforderungen der Praxis.

Es wurde nun gefunden, daß neue N-substituierte aromatische Brenztraubensäureamide der Formel

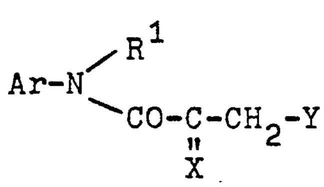

I,

worin

Ar 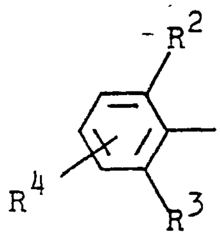 oder 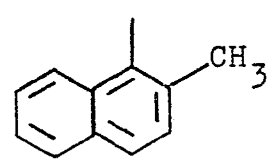 ,

$R^1$ $-CH-Z$, $CH_3$ (an CH), 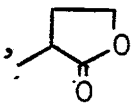 , 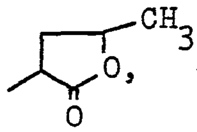 oder $-CH=C(CN)_2$,

X O, $N-NR^5R^6$, $N-NH-CO-NR^5R^6$, $N-NH-CS-NR^5R^6$, $N-O-R^7$

Y H oder Br

Z $CO_2CH_3$, $CH(OCH_3)_2$, $CONH-NR^5R^6$, $CO-NH-NH-CS-NR^5R^6$

oder $CH_2-CH_2-$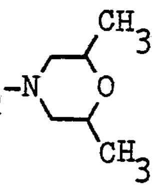 ,

$R^2$ $CH_3$

$R^3$ H, $CH_3$, $C_2H_5$ oder Cl

$R^4$ H, $CH_3$, $C_6H_5$ oder Br

$R^5$ und $R^6$ unabhängig voneinander H, $CH_3$, $C_2H_5$ oder $C_6H_5$ und

$R^7$ H, $CH_3$, $C_2H_5$, $-CH_2-CH=CH_2$, ggf. substituiertes Benzyl, $-CH_2-O-CO-CH_3$, $CH_2-CO-O-CH_3$ oder $-CO-CH_3$ bedeutet.

eine gute fungizide Wirkung besitzen.

Die neuen N-substituierten Brenztraubensäureamide besitzen im 2-Kohlenstoff des Butyrolacton-Rings ein Asymmetriezentrum; zudem können durch die eingeschränkte Drehbarkeit der C-N-Bindung im aromatischen Aminteil bei Raumtemperatur stabile Rotamere (Atropisomere) entstehen. Außerdem besteht bei einigen Derivaten, insbesondere bei Oximen, die Möglichkeit der syn-anti-Stereoisomerie. Mit üblichen Methoden können die opt. reinen Enantiomeren bzw. die Diastereomeren, letztere z.T. schon durch Umkristallisieren, getrennt werden. Die vorliegende Erfindung umfaßt sowohl die einzelnen Isomeren als auch die entsprechenden Gemische.

Die neuen Verbindungen können hergestellt werden z.B. durch Umsetzung des entsprechenden Amins, das den Brenztraubensäurerest nicht enthält, mit einem entsprechenden Brenztraubensäurechlorid oder einem gemischten Säureanhydrid, das einen Brenztraubensäurerest enthält, wobei in einem Lösungs- oder Verdünnungsmittel unter Zusatz einer Base und eines Reaktionsbeschleunigers gearbeitet werden kann.

Lösungs- oder Verdünnungsmittel sind z.B. aliphatische oder aromatische Kohlenwasserstoffe, wie Pentan, Cyclohexan, Petrolether, Benzol, Toluol oder Xylole; Halogenkohlenwasserstoffe, beispielsweise Methylenchlorid, 1,2-Dichlorethan, Chlorbenzol; Ketone, wie Aceton und Methylethylketon; Ether, wie Diethylether, Dimethoxyethan, Tetrahydrofuran oder Dioxan; Ester, wie Essigsäureethylester, ferner Acetonitril, Dimethylsulfoxid, Dimethylformamid und Acetanhydrid.

Anorganische oder organische Basen sind z.B. Alkali- und Erdalkalicarbonate, wie Natrium- oder Kaliumhydrogencarbonat, Natrium- oder Kaliumcarbonat, Calcium- oder Bariumcarbonat; Amine, wie Triethylamine, N,N-Dimethylanilin, N-Methylpiperidin oder Pyridin.

Reaktionsbeschleuniger sind z.B. Metallhalogenide, wie Natriumbromid oder Kaliumiodid, Azole wie Imidazol oder 1,2,4-Triazol; Pyridine, wie 4-Dimethylaminopyridin oder Dimethylformamid.

Man kann statt des Brenztraubensäurechlorids auch die freie Brenztraubensäure zusammen mit anorganischen oder organischen Säurechloriden und einer Base und einem Lösungs- oder Verdünnungsmittel verwenden.

Anorganische oder organische Säurechloride sind z.B. Thionylchlorid, Phosphortrichlorid, Phosphoroxychlorid, Phosphorpentachlorid, Phosphortribromid, Acetylchlorid oder p-Toluolsulfonsäurechlorid.

Man kann auch das Brenztraubensäureamid, das den Propionsäuremethylester-Rest enthält, mit Alkalihydroxid zum entsprechenden Alkalisalz des Propionsäurederivats verseifen und das Salz an der C=O-Gruppe des Brenztraubensäure-Restes mit Hydrazin- oder Hydroxylaminderivaten unter Wasserabspaltung in Anwesenheit eines Puffers in einem Lösungs- oder Verdünnungsmittel umsetzen und danach den Propionsäurerest mit Methanol zum Propionsäuremethylester--Rest in üblicher Weise verestern.

Ein Puffer ist beispielsweise Natriumacetat, Kaliumacetat, Natrium- oder Kaliummono- oder -diphosphat oder Natriumborat.

Man kann ferner Verbindungen, die dem Anspruch 1 entsprechen, aber anstelle des Brenztraubensäurerestes den Rest alpha-Hydroxypropionsäure enthalten, durch Oxydation in das entsprechende Brenztraubensäurederivat überführen. Verbindungen mit dem Rest der alpha-Hydroxypropionsäure werden beschrieben in der DE-OS 29 17 893 und DE-OS 29 02 861.

Die Umsetzung des Amins mit dem Brenztraubensäurechlorid wird bevorzugt.

Die nachfolgenden Beispiele dienen zur näheren Erläuterung der Erfindung, ohne dieselbe einzuschränken. Wenn nicht anders vermerkt, ist bei Nennung eines Wirkstoffs der Formel I stets das racemische Gemisch möglicher Isomerer gemeint.

Beispiel 1

Herstellung von N-(2-Methyl-1-naphthyl)-N-(alpha-butyro-gamma-lactonyl)-brenztraubensäure-amid (Verbindung Nr. 1)

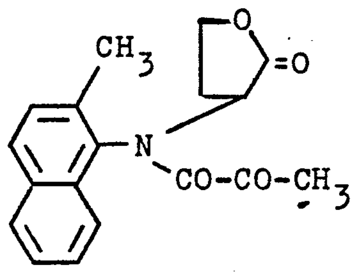

33,7 g N-(alpha-Butyro-gamma-lactonyl)-2-methyl-1-naphthylamin und 15,3 g Brenztraubensäurechlorid werden in 250 ml trockenem Toluol 3h bei 70°C erhitzt, wobei man einen leichten Stickstoffstrom einleitet. Nach dem Abkühlen saugt man vom Niederschlag ab, extrahiert das Filtrat dreimal mit Wasser, trocknet und engt ein. Den Rückstand löst man aus Isopropanol um, wobei Kristalle der Verbindung Nr. 1 vom Schmp. 160°C erhalten werden.

Beispiel 2

Herstellung von N-(2,6-Dimethyl-phenyl)-N-[(1-methoxycarbonyl)-1-ethyl]-2-[O-(2,6-dichlor-benzyl)-oximino]-brenztraubensäure-amid (Verbindung Nr. 22)

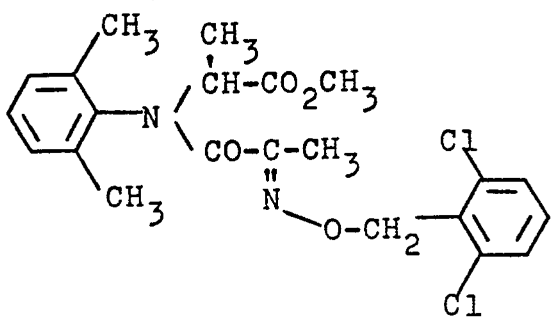

22,9 g der Verbindung Nr. 12 werden mit 27,7 g Hydroxylamin-O-(2,6-dichlorbenzyl)-ether-hydrochlorid und 7,8 g

Kaliumacetat in 250 ml trockenem Ethanol 12 h unter Rückfluß erhitzt. Danach engt man ein, nimmt in Methylenchlorid auf, schüttelt nacheinander mit Wasser, verd. Natronlauge und wiederum Wasser aus, trocknet, entfernt das Methylenchlorid und kristallisiert den Rückstand aus Ethanol/Wasser 1:2 um. Dabei fallen Kristalle der Verbindung Nr. 22 vom Schmp. 76 bis 78°C an.

Beispiel 3

Herstellung von N-(2,6-Dimethyl-phenyl)-N-[(1-methoxycarbonyl)-1-ethyl]-2-[(N-phenyl)-hydrazimino]-brenztraubensäure-amid (Verbindung Nr. 26)

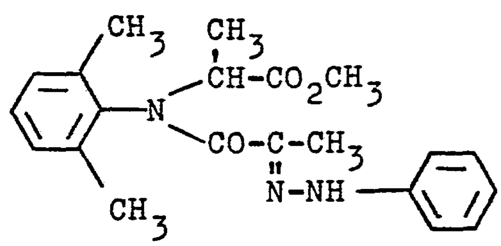

22,9 g des in Beispiel 2 als Ausgangsprodukt verwendeten Brenztraubensäureamids, 10,8 g Phenylhydrazin und 7,8 g Kaliumacetat erhitzt man 12 h bei 60°C. Danach wird eingeengt, in Methylenchlorid aufgenommen, mit verd. Natronlauge und Wasser gewaschen, getrocknet und vom Methylenchlorid abdestilliert. Der Rückstand wird säulenchromatographiert. Man erhält Kristalle der Verbindung Nr. 26 vom Schmp. 108 bis 110°C.

Beispiel 4

N-(2,6-Dimethyl-phenyl)-N-[1-(thiosemicarbazido-carbonyl)-1-ethyl]-brenztraubensäure-amid (Verbindung Nr. 31)

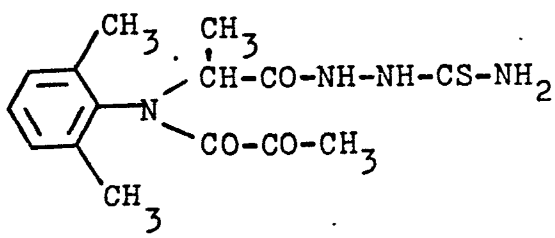

27,7 g der Verbindung Nr. 12, 13,7 g Thiosemicarbazid und 9,8 g Kaliumacetat werden in 200 ml Ethanol/Wasser-Gemisch 5 h unter Rückfluß erhitzt. Nach Abkühlen saugt man vom ausgefallenen Niederschlag ab, der gelöst und an einer Aluminiumoxidsäule chromatographiert wird. Man erhält Kristalle der Verbindung Nr. 31 vom Schmp. 180 bis 183°C.

Beispiel 5

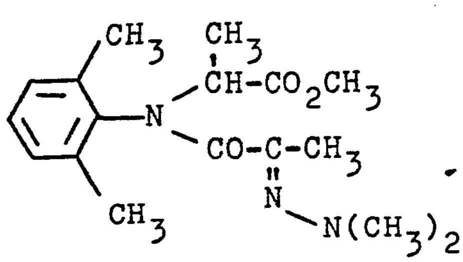

N-(2,6-Dimethyl-phenyl)-N-[(1-methoxycarbonyl)-1-ethyl]-2--[(N,N-dimethyl)-hydrazimino]-brenztraubensäure-amid (Verbindung Nr. 25)

27,7 g der Verbindung Nr. 12 werden nach üblichen Methoden zum Kaliumsalz des Propionsäurederivats verseift. Das Salz erhitzt man zusammen mit 6,0 g asym.-Dimethylhydrazin 24 h unter Rückfluß, engt nach Abkühlen ein, stellt die Lösung mit Säure auf pH 3 ein und extrahiert mit Dichlormethan. Nach Trocknen und Abtrennen des Lösungsmittels erhält man einen festen Rückstand, der durch Kochen in Methanol unter Zusatz katalytischer Mengen Bortrifluorid-Etherat in den

Methylester überführt wird. Reinigung durch Säulenchromatographie führt zur Reinsubstanz, die als gelbliches Öl (Verbindung Nr. 25) anfällt.

Nach den genannten Methoden können folgende Verbindungen der Formel I hergestellt werden:

Tabelle

| Lfd. Nr. | Ar | $R^1$ | X | Y | phys. Daten Schmp. °C | IR-Banden ($cm^{-1}$) (fingerprint) |
|---|---|---|---|---|---|---|
| 1 | 2-Methyl-naphthyl | alpha-Butyro-gamma-lactonyl | O | H | 160 | |
| 2 | 2,6-Dimethyl-phenyl | " | O | H | 142 | |
| 3 | 2-Methyl-phenyl | " | O | H | 93- 95 | |
| 4 | 2-Clor-6-methyl-phenyl | " | O | H | 110-112 | |
| 5 | 2,5,6-Trimethyl-phenyl | " | O | H | 120-125 | |
| 6 | 2-Methyl-6-ethyl-phenyl | " | O | H | 103 | |
| 7 | 2-Methyl-naphthyl | " | N-OMethyl | H | 57 | } Stereoisomere |
| 8 | 2-Methyl-naphthyl | " | N-OMethyl | H | 181 | } Stereoisomere |
| 9 | 2,6-Dimethyl-phenyl | " | N-OMethyl | H | 102-104 | |
| 10 | 2,6-Dimethyl-phenyl | " | H<br>N-N-CS-N-dimethyl | H | 126-128 | |
| 11 | 2-Methyl-naphthyl | 1-(1-Methoxy-carbonyl)-ethyl | O | H | 136 | |
| 12 | 2,6-Dimethyl-phenyl | " | O | H | 60 | |

| Lfd. Nr. | Ar | $R^1$ | X | Y | phys. Daten Schmp. °C | IR-Banden ($cm^{-1}$) (fingerprint) |
|---|---|---|---|---|---|---|
| 13 | 2-Chlor-6-methyl-phenyl | 1-(1-Methoxy-carbonyl)-ethyl | O | H | Öl | 1468,1455,1356,1203, 1172,788 |
| 14 | 2,4,6-Trimethyl-phenyl | " | O | H | 87 | |
| 15 | 2-Methyl-6-ethyl-phenyl | " | O | H | Öl | 1455,1345,1200,1165 |
| 16 | 2,6-Dimethyl-4-brom-phenyl | " | O | H | 88 | |
| 17 | 2,6-Dimethyl-(4-phenyl)-phenyl | " | O | H | Öl | 1345,1205,1165,765, 703 |
| 18 | 2,6-Dimethyl-phenyl | " | N-OMethyl | H | 58- 60 | |
| 19 | " | " | N-OEthyl | H | 68- 70 | |
| 20 | " | " | N-O-$CH_2$-CH=$CH_2$ | H | Öl | 1195,1173,1022,992, 928,775 |
| 21 | " | " | N-O-Benzyl | H | 65- 68 | |
| 22 | " | " | N-O-(2,6-Di-chlor-benzyl) | H | 76- 78 | |
| 23 | " | " | N-O-(2,4-Di-chlor-benzyl) | H | | |
| 24 | " | " | N-OH | H | 134-135 | |
| 25 | " | " | N-NDimethyl | H | Öl | 1450(breit),1375, 1345,1200,1170,772 |
| 26 | " | " | N-NH-Phenyl | H | 108-110 | |

| Lfd. Nr. | Ar | $R^1$ | X | Y | phys. Daten Schmp. °C | phys. Daten IR-Banden ($cm^{-1}$) (fingerprint) |
|---|---|---|---|---|---|---|
| 27 | 2,6-Dimethyl-phenyl | 1-(1-Methoxy-carbonyl)-ethyl | $N-NH-CONH_2$ | H | 236-238 | |
| 28 | " | " | $N-OCH_2OCOMethyl$ | H | | |
| 29 | " | " | $N-O-CH_2COOMethyl$ | H | | |
| 30 | " | CH(Methyl)CO-N(H)-Ndimethyl | O | H | 103-106 | |
| 31 | " | $CH(Methyl)CO-N(H)-N(H)-CS-NH_2$ | O | H | 180-183 | |
| 32 | " | CH(Methyl)CO-N(H)-N(H)-CS-N(H)-methyl | O | H | 243-245 | |
| 33 | " | CH(Methyl)CO-N(H)-N(H)-CS-N(H)-ethyl | O | H | 176-178 | |
| 34 | " | CH(Methyl)CO-N(H)-N(H)-CS-N-dimethyl | O | H | | |
| 35 | " | $CH(Methyl)(CH_2)_2$- 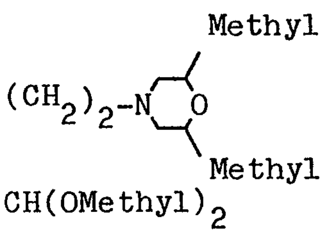 | O | H | Öl | 1462,1376,1179,1165, 1145,1079,787 |
| 36 | 2-Chlor-6-methyl-phenyl | $CH(Methyl)CH(OMethyl)_2$ | O | H | Öl | 1455(br),1382,1178, 1150,1130(br),1025(br), 778 [br = breit] |

| Lfd. Nr. | Ar | $R^1$ | X | Y | phys. Daten Schmp. °C | IR-Banden ($cm^{-1}$) (fingerprint) |
|---|---|---|---|---|---|---|
| 37 | 2,6-Dimethyl-phenyl | $CH=C(CN)_2$ | O | H | | |
| 38 | " | $CH(Methyl)CO-\overset{H}{N}-\overset{H}{N}-CO-NH_2$ | O | H | 232-237 | |
| 39 | " | 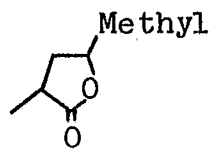 | O | H | 110 | |
| 40 | " | $CH(Methyl)CO_2Methyl$ | O | Br | 80 | |
| 41 | " | 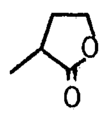 | O | Br | | |
| 42 | 2-Methyl-naphthyl | $CH(Methyl)CO_2Methyl$ | O | Br | öl | 1449,1425 ,1365, 1345,1198(br),1139, 818,785,748 |
| 43 | 4-Brom-2,6-di-methyl-phenyl | $CH(Methyl)CO_2Methyl$ | O | Br | öl | 1473(br),1455(br),1378, 1337,1275,1246,1204(br), 1180,859,830 |
| 44 | 2-Methyl--naphthyl | 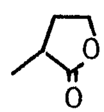 | O | Br | öl | 1405,1377,1170(br), 1120(br),1025,818, 780,748 |
| 45 | 2,3,6-Trimethyl-phenyl | 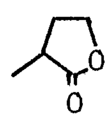 | O | Br | 86 | |

| Lfd. Nr. | Ar | $R^1$ | X | Y | phys. Daten Schmp. °C | IR-Banden ($cm^{-1}$) (fingerprint) |
|---|---|---|---|---|---|---|
| -46 | 2,6-Dimethyl-phenyl | CH(Methyl)$CO_2$Methyl | N-O-CO-Methyl | H | öl | 1473,1456,1435,1380, 1367,1201(br),1185(br), 990(br),932,780 |
| 47 | 2-Methyl--naphthyl | CH(Methyl)$CO_2$Methyl | N-O-(2,6-Di-chlor-benzyl) | H | öl | 1437,1391,1366,1197, 1180,1022,764,768 |
| 48 | 2,6-Dimethyl--phenyl | 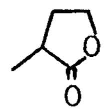 | " | H | öl | 1438(stark),1225(br), 1197,1168(br),1027(stark, br),995,780,768 |
| 49 | 2-Methyl--naphthyl | CH(Methyl)$CO_2$Methyl | N-O-Ethyl | H | öl | 1455,1391(br),1351, 1200(br),1178,1141, 1047,818,788,750 |
| 50 | " | 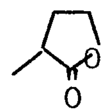 | N-O-Benzyl | H | 103-106 | |
| -51 | " | CH(Methyl)$CO_2$Methyl | " | H | öl | 1454,1392,1365,1198(br), 1178,1015(br),817,748 |
| -52 | 2,6-Dimethyl-phenyl | 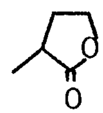 | N-O-Ethyl | H | öl | 1474,1444,1413,1381, 1341,1224,1166,1042(br), 775 [br=breit] |

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Erysiphe graminis an Getreide, Uncinula necator an Reben, Podophaera leucotricha an Äpfeln, Sphaerotheca fuliginea an Rosen, Erysiphe cichoracearum an Gurken. Die fungiziden Mittel enthalten 0,1 bis 95 % (Gew.%) Wirkstoff, vorzugsweise 0,5 bis 90 %. Die Aufwandmengen liegen je nach Art des gewünschten Effektes zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Darüber hinaus sind viele der neuen Verbindungen systemisch wirksam, so daß über die Wurzelbehandlung auch ein Schutz oberirdischer Pflanzenteile möglich ist.

Die neuen Wirkstoffe können auch zusammen mit anderen Wirkstoffen, z.B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf,

d.h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt:

Manganethylenbisdithiocarbamat
Mangen-Zinkethylenbisdithiocarbamat
Ammoniak-Komplex von Zink-(N,N-ethylen-bis-dithiocarbamat)
N-Trichlormethylthio-tetrahydrophthalimid
N-Trichlormethyl-phthalimid
5-Ethoxy-3-trichlormethyl-1,2,3-thiadiazol
2-Methoxycarbonylamino-benzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion.

Die folgende Liste von Fungiziden, mit denen die neuen Verbindungen kombiniert werden können, soll die Kombinationsmöglichkeiten erläutern, nicht aber einschränken.

Fungizide, die mit den erfindungsgemäßen Wirkstoffen kombiniert werden können, sind beispielsweise:

Dithiocarbamate und deren Derivate, wie
Ferridimethyldithiocarbamat
Zinkdimethyldithiocarbamat
Zinkethylenbisdithiocarbamat
Tetramethylthiuramdisulfide
Zink-(N,N-propylen-bis-dithiocarbamat)
Ammoniak-Komplex von Zink-(N,N'-propylen-bis-dithiocarbamat)
und
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat

heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-
-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-
-dioxid
2,3-Dihydro-5-carboxanilido-6-methyl-1,4-oxathiin
2-(Furyl-(2))-benzimidazol
Piperazin-1,4-diylbis-(1-(2,2,2-trichlor-ethyl)-formamid
2-(Thiazolyl-(4)-benzimidazol
5-Butyl-2-dimethylamino-4-hydroxy-6-methyl-pyrimidin
Bis-(p-chlorphenyl)-3-pyridinmethanol

1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-glutarimid
Hexachlorbenzol
N-Dichlorfluormethylthio-N',N'-dimethyl-N-phenyl-schwefelsäurediamid
2,5-Dimethyl-furan-3-carbonsäureanilid
2-Methyl-benzoesäure-anilid
2-Jod-benzoesäure-anilid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
1-(4-Chlorphenoxy)-3,3-dimethyl-1-(1H-1,2,4-triazol-1-yl)--2-butanon
1-(4-Chlorphenoxy)-3,3-dimethyl-1-(1H-1,2,4-triazol-1-yl)--2-butanol
alpha-(2-Chlorphenyl)-alpha-(4-chlorphenyl)-5-pyrimidin--methanol.

Die neuen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten durch Versprühen, Vernebeln, Verstäuben, Verstreuen, Beizen oder Gießen angewendet. Die Anwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfrak-

tionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht: Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol-, Nonylphenol, Alkylphenolpolyglykolether, Tributylphenylpolyglykolether, Alkalarylpolyetheralkohole, Isotridecylalkohol, Fettalkoholethylenoxid-Kondensate, ethoxyliertes Rizinusöl, Polyoxyäthylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatommeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Für die folgenden Versuche wurden als bekannte Vergleichswirkstoffe die folgenden Verbindungen verwendet:

N-Trichlormethylthio-phthalimid (Verbindung A)
N-Trichlormethylthio-tetrahydrophthalimid (Verbindung B)
Zink-ethylen-1,2-bis-dithiocarbamat (Verbindung C)

Versuch 1

Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruchs abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß beispielsweise die Verbindungen Nr. 1, 2, 11 und 12 bei Anwendung als 0,025 %ige Spritzbrühe eine bessere fungizide Wirkung (beispielsweise 100 %) zeigen als der bekannte Wirkstoff A (beispielsweise 90 %).

Versuch 2

Wirksamkeit gegen Phytophthora infestans an Tomaten

Blätter von Topfpflanzen der Sorte "Große Fleischtomate" werden mit wäßriger Spritzbrühe, die 80 % Wirkstoff und 20 % Emulgiermittel in der Trockensubstanz enthält, besprüht. Nach dem Antrocknen des Spritzbelages werden die

Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise die Verbindungen Nr. 1, 2 und 22 bei Anwendung als 0,025 %ige Spritzbrühe eine bessere fungizide Wirkung zeigen (beispielsweise 90 %) als die bekannten Wirkstoffe B und C (beispielsweise 70 %).

Beispiele für Zubereitungen sind:

I. Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung 11 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser er--hält man eine wäßrige Dispersion.

IV. 20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser, erhält man eine wäßrige Dispersion.

V. 80 Gewichtsteile der Verbindung 12 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammer--mühle vermahlen. Durch feines Verteilen der Mischung in Wasser erhält man eine Spritzbrühe.

VI. 3 Gewichtsteile der Verbindung 2 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 2 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung 11 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineral-öls innig vermischt. Man erhält eine stabile ölige Dispersion.

## Patentansprüche

1. N-substituiertes Brenztraubensäureamid der Formel I

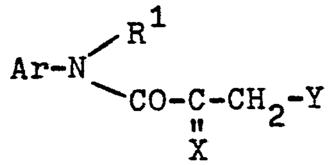 I,

worin

Ar 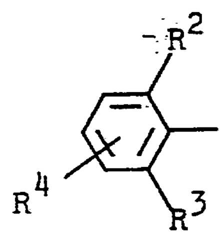 oder 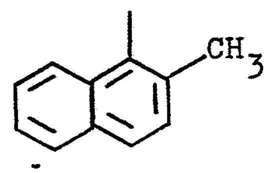 ,

$R^1$ 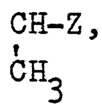 , 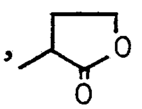 , 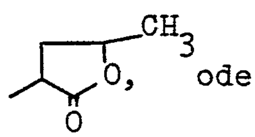 oder $-CH=C(CN)_2$,

X O, $N-NR^5R^6$, $N-NH-CO-NR^5R^6$, $N-NH-CS-NR^5R^6$, $N-O-R^7$

Y H oder Br

Z $CO_2CH_3$, $CH(OCH_3)_2$, $CONH-NR^5R^6$, $CO-NH-NH-CS-NR^5R^6$ oder $CH_2-CH_2-$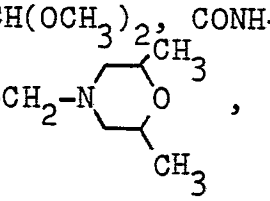 ,

$R^2$ $CH_3$

$R^3$ H, $CH_3$, $C_2H_5$ oder Cl

$R^4$ H, $CH_3$, $C_6H_5$ oder Br

$R^5$ und $R^6$ unabhängig voneinander H, $CH_3$, $C_2H_5$ oder $C_6H_5$ und

$R^7$ H, $CH_3$, $C_2H_5$, $-CH_2-CH=CH_2$, ggf. substituiertes Benzyl, $-CH_2-O-CO-CH_3$, $CH_2-CO-O-CH_3$ oder $-CO-CH_3$ bedeutet.

2. Verbindung gemäß Anspruch 1, <u>dadurch gekennzeichnet</u>, daß

Ar 3-Methyl-1-naphthyl oder 2,6-Dimethylphenyl
$R^1$ alpha-Butyro-gamma-lactonyl oder 1-Methoxycarbonyl-1-ethyl
X Sauerstoff oder O-2,6-Dichlorbenzyl-oximino und
Y Wasserstoff bedeutet.

3. Fungizide Mittel, die eine Verbindung gemäß Anspruch 1 enthalten.

4. Fungizid, enthaltend eine Verbindung gemäß Anspruch 1, <u>dadurch gekennzeichnet</u>, daß

Ar 2-Methyl-1-naphthyl oder 2,6-Dimethylphenyl
$R^1$ alpha-Butyro-gamma-lactonyl oder 1-Methoxycarbonyl-1-ethyl
X Sauerstoff oder O-2,6-Dichlorbenzyl-oximino und
Y Wasserstoff bedeutet.

5. Verfahren zur Bekämpfung von Pilzen, <u>dadurch gekennzeichnet</u>, daß man eine fungizid wirksame Menge einer Verbindung gemäß Anspruch 1 auf die Pilze oder die vor Pilzbefall zu schützenden Pflanzen, Flächen oder Saatgüter einwirken läßt.

6. Verfahren zur Herstellung einer Verbindung gemäß Anspruch 1, dadurch gekennzeichnet, daß man ein Amin der Formel

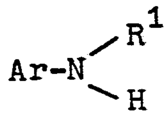

II,

worin Ar und $R^1$ die in Anspruch 1 angegebenen Bedeutungen haben,

a) mit einem Säurechlorid der Formel

$$Y - CH_2 - \underset{\underset{X}{\parallel}}{C} - CO - Cl \qquad III,$$

b) mit einem Säureanhydrid der Formel

$$Y - CH_2 - \underset{\underset{X}{\parallel}}{C} - CO - O - CO - R^8 \qquad IV,$$

wobei $R^8$ die Bedeutung $C_1$-$C_4$-Alkyl oder O-$C_1$-$C_4$-Alkyl hat, und Y und X die im Anspruch 1 genannten Bedeutungen haben,

gegebenenfalls in einem Lösuns- oder Verdünnungsmittel, gegebenenfalls unter Zusatz einer anorganischen oder organischen Base und gegebenenfalls unter Zusatz eines Reaktionsbeschleunigers bei Temperaturen zwischen 0 und 120°C umsetzt.

7. Verfahren zur Herstellung von Verbindungen gemäß Anspruch 1, dadurch gekennzeichnet, daß man ein Amin der Formel II mit einer Carbonsäure der Formel

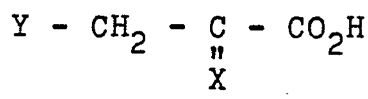

V,

wobei X und Y die im Anspruch 1 genannten Bedeutungen haben, in Anwesenheit von anorganischen oder organischen Säurechloriden und einer anorganischen oder organischen Base in einem Lösungs- oder Verdünnungsmittel bei Temperaturen zwischen -20 und +120°C umsetzt.

8. Verfahren zur Herstellung von Verbindungen gemäß Anspruch 1, dadurch gekennzeichnet, daß man ein Amid der Formel I in der $R^1 = CH(CH_3)CO_2CH_3$ und X = O und Y = H bedeutet, mit Alkalihydroxid zu Salzen der Formel VI verseift,

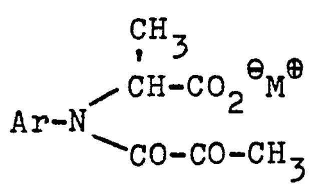

VI,

$M^{\oplus}$ = Natrium oder Kalium

diese mit einer Verbindung der Formel $XH_2$ oder $XH_2 \cdot HA$, wobei X die in Anspruch 1 angegebene Bedeutung außer O hat und A einen anorganischen oder organischen Säurerest bedeutet, unter Wasserabspaltung, gegebenenfalls in Anwesenheit einer Lösung eines anorganischen oder organischen Puffers, in einem Lösungs- oder Verdünnungsmittel bei Temperaturen zwischen +20 und +150°C umsetzt, danach mit anorganischer oder organischer Säure die Säure der Formel VIIa freisetzt und diese mit Methanol in üblicher Weise zum Ester VIIb umsetzt

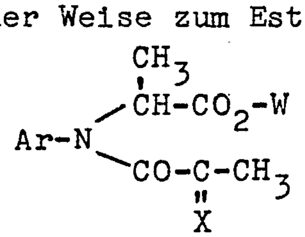

VII a: W = H
b: $W = CH_3$

wobei Ar und X die im Anspruch 1 genannten Bedeutungen haben.

9. Verfahren zur Herstellung von Verbindungen gemäß Anspruch 1 mit der Bedeutung X = O, dadurch gekennzeichnet, daß man eine Verbindung der Formel VIII

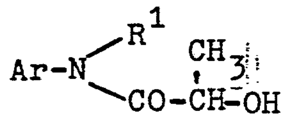

VIII,

in der Ar und $R^1$ die im Anspruch 1 angegebenen Bedeutungen haben, umsetzt mit einem Oxidationsmittel aus der Gruppe $Na_2Cr_2O_7/H_2SO_4/H_2O$, $K_2Cr_2O_7/H_2SO_4/H_2O$, $CrO_3/H_2O$/Aceton, $CrO_3$/Pyridin oder aktiviertes Mangandioxid.

Patentansprüche (für Österreich)

1. Fungizid, enthaltend ein N-substituiertes Brenztraubensäureamid der Formel I

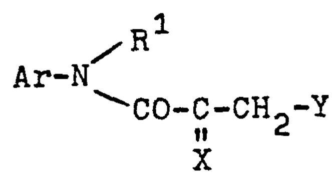 I,

worin

Ar 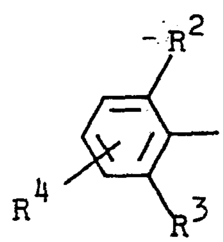 oder 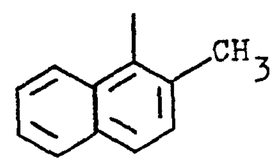 ,

$R^1$ 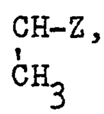 , 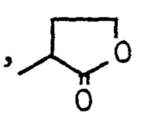 , 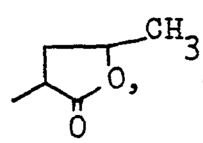 oder $-CH=C(CN)_2$,

X O, $N-NR^5R^6$, $N-NH-CO-NR^5R^6$, $N-NH-CS-NR^5R^6$, $N-O-R^7$

Y H oder Br

Z $CO_2CH_3$, $CH(OCH_3)_2$, $CONH-NR^5R^6$, $CO-NH-NH-CS-NR^5R^6$ oder $CH_2-CH_2-$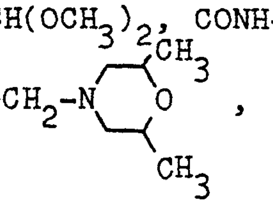 ,

$R^2$ $CH_3$

$R^3$ H, $CH_3$, $C_2H_5$ oder Cl

$R^4$ H, $CH_3$, $C_6H_5$ oder Br

$R^5$ und $R^6$ unabhängig voneinander H, $CH_3$, $C_2H_5$ oder $C_6H_5$ und

$R^7$ H, $CH_3$, $C_2H_5$, $-CH_2-CH=CH_2$, ggf. substituiertes Benzyl, $-CH_2-O-CO-CH_3$, $CH_2-CO-O-CH_3$ oder $-CO-CH_3$ bedeutet.

2. Fungizid, enthaltend eine Verbindung gemäß Anspruch 1, <u>dadurch gekennzeichnet</u>, daß

Ar 2-Methyl-1-naphthyl oder 2,6-Dimethylphenyl
$R^1$ alpha-Butyro-gamma-lactonyl oder 1-Methoxycarbonyl-1-ethyl
X Sauerstoff oder O-2,6-Dichlorbenzyl-oximino und
Y Wasserstoff bedeutet.

3. Verfahren zur Bekämpfung von Pilzen, <u>dadurch gekennzeichnet</u>, daß man eine fungizid wirksame Menge einer Verbindung gemäß Anspruch 1 auf die Pilze oder die vor Pilzbefall zu schützenden Pflanzen, Flächen oder Saatgüter einwirken läßt.

4. Verfahren zur Herstellung einer Verbindung gemäß Anspruch 1, <u>dadurch gekennzeichnet</u>, daß man ein Amin der Formel

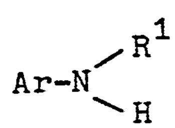

II,

worin Ar und $R^1$ die in Anspruch 1 angegebenen Bedeutungen haben,

a) mit einem Säurechlorid der Formel

$$Y - CH_2 - \underset{\overset{\Vert}{X}}{C} - CO - Cl \qquad III,$$

b) mit einem Säureanhydrid der Formel

$$Y - CH_2 - \underset{\overset{\Vert}{X}}{C} - CO - O - CO - R^8 \qquad IV,$$

wobei $R^8$ die Bedeutung $C_1$-$C_4$-Alkyl oder O-$C_1$-$C_4$--Alkyl hat, und Y und X die im Anspruch 1 genannten Bedeutungen haben,

gegebenenfalls in einem Lösuns- oder Verdünnungsmittel, gegebenenfalls unter Zusatz einer anorganischen oder organischen Base und gegebenenfalls unter Zusatz eines Reaktionsbeschleunigers bei Temperaturen zwischen 0 und 120°C umsetzt.

5. Verfahren zur Herstellung von Verbindungen gemäß Anspruch 1, <u>dadurch gekennzeichnet</u>, daß man ein Amin der Formel II mit einer Carbonsäure der Formel

$$Y - CH_2 - \underset{\underset{X}{\|}}{C} - CO_2H \qquad V,$$

wobei X und Y die im Anspruch 1 genannten Bedeutungen haben, in Anwesenheit von anorganischen oder organischen Säurechloriden und einer anorganischen oder organischen Base in einem Lösungs- oder Verdünnungsmittel bei Temperaturen zwischen -20 und +120°C umsetzt.

6. Verfahren zur Herstellung von Verbindungen gemäß Anspruch 1, <u>dadurch gekennzeichnet</u>, daß man ein Amid der Formel I in der $R^1 = CH(CH_3)CO_2CH_3$ und X = O und Y = H bedeutet, mit Alkalihydroxid zu Salzen der Formel VI verseift,

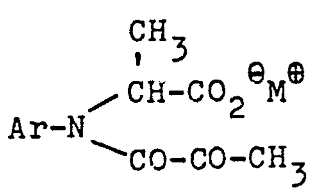

VI,

$M^{\oplus}$ = Natrium oder Kalium

diese mit einer Verbindung der Formel $XH_2$ oder $XH_2$ . HA, wobei X die in Anspruch 1 angegebene Bedeutung außer O hat und A einen anorganischen oder organischen Säurerest bedeutet, unter Wasserabspaltung, gegebenenfalls in Anwesenheit einer Lösung eines anorganischen oder organischen Puffers, in einem Lösungs- oder Verdünnungsmittel bei Temperaturen zwischen +20 und +150°C umsetzt, danach mit anorganischer oder organischer Säure die Säure der Formel VIIa freisetzt und diese mit Methanol in üblicher Weise zum Ester VIIb umsetzt

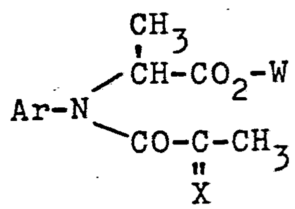

VII a: W = H
b: W = $CH_3$

wobei Ar und X die im Anspruch 1 genannten Bedeutungen haben.

7. Verfahren zur Herstellung von Verbindungen gemäß Anspruch 1 mit der Bedeutung X = O, <u>dadurch gekennzeichnet</u>, daß man eine Verbindung der Formel VIII

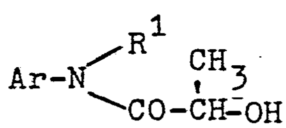

VIII,

in der Ar und $R^1$ die im Anspruch 1 angegebenen Bedeutungen haben, umsetzt mit einem Oxidationsmittel aus der Gruppe $Na_2Cr_2O_7/H_2SO_4/H_2O$, $K_2Cr_2O_7/H_2SO_4/H_2O$, $CrO_3/H_2O$/Aceton, $CrO_3$/Pyridin oder aktiviertes Mangandioxid.

Europäisches Patentamt

# EUROPÄISCHER RECHERCHENBERICHT

0075139 Nummer der Anmeldung

EP 82 10 7948.0

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | EP - A1 - 0 029 996 (BASF)<br>* Ansprüche 1, 2; Zusammenfassung *<br>-- | 1,4 |
| A | EP - A1 - 0 018 510 (CIBA-GEIGY)<br>* Ansprüche 1, 11 *<br>-- | 1,4 |
| D,A | DE - A1 - 2 917 893 (CIBA-GEIGY)<br>* Ansprüche 1, 5 *<br>-- | 1,4 |
| D,A | DE - A1 - 2 902 861 (CIBA-GEIGY)<br>* Ansprüche 1, 5 *<br>-- | 1,4 |
| P,A | Patent Abstracts of Japan<br>Band 6, Nr. 68, 30. April 1982<br>& JP - A - 57 - 7454<br>-- | |
| A | Chemical Abstracts Band 89, Nr. 25, 18. Dezember 1978<br>Columbus, Ohio, USA<br>W. STEGLICH et al. " Activated carboxylic acid derivatives. II. A simple synthesis of 2-oxocarboxylic acid amides, N-(2-oxoacyl)amino acid esters and 2-oxocarboxylic acid hydrazides"<br>Seite 545, Spalte 2, Abstract Nr. 214864w<br>& Synthesis, Nr. 8, 1978, Seiten 622 bis 624<br>---- | |

**KLASSIFIKATION DER ANMELDUNG (Int. Cl.³)**

C 07 D 307/32
C 07 D 265/30
C 07 C 103/38
C 07 C 103/48
C 07 C 109/087
C 07 C 109/18
C 07 C 121/30
C 07 C 131/00
C 07 C 133/02
C 07 C 133/04
C 07 C 159/00
A 01 N 35/10
A 01 N 37/22
A 01 N 37/36

**RECHERCHIERTE SACHGEBIETE (Int. Cl.³)**

A 01 N 35/10
A 01 N 37/22
A 01 N 37/36
A 01 N 43/08
A 01 N 43/84
A 01 N 47/34
C 07 C 103/38
C 07 C 103/48
C 07 C 109/087
C 07 C 109/18
C 07 C 121/30
C 07 C 131/00
C 07 C 133/02
C 07 C 133/04

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 29-11-1982 | PHILLIPS |

Europäisches Patentamt

**EUROPÄISCHER RECHERCHENBERICHT**

0075139

Nummer der Anmeldung

EP 82 10 7948.0

- Seite 2 -

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | A 01 N 43/08<br>A 01 N 43/84<br>A 01 N 47/34 |
| | | | RECHERCHIERTE SACHGEBIETE (Int. Cl.³) |
| | | | C 07 C 159/00<br>C 07 D 265/30<br>C 07 D 307/32 |